# EUROPÄISCHE PATENTANMELDUNG

(11) **EP 1 350 486 A1**
(43) Veröffentlichungstag der Anmeldung: **08.10.2003**
(21) Anmeldenummer: 03006677.3
(22) Anmeldetag: 26.03.2003
(51) Int. Cl.: A61F 2/36

(54) **Endoprothese**

(30) Priorität: 04.04.2002 DE 10214808
(71) Anmelder: Aesculap AG & Co. KG, 78532 Tuttlingen (DE)
(72) Erfinder: Reu, Gerhard, 78532 Tuttingen (DE); Stern, Andreas, 78052 Villingen-Schwenningen Weilersbach (DE); Wallstein, Stefan, 78532 Tuttingen (DE)
(74) Vertreter: Böhme, Ulrich, Dr. Dipl.-Phys.

(57) **Zusammenfassung**

Um bei einer Endoprothese mit einem Verankerungsteil (1) und einem Verbindungsteil (4), bei der das Verbindungsteil (4) mit einem zapfenförmigen Einsteckende (5) in eine sacklochförmige Aufnahmeöffnung (2) am Verankerungsteil (1) einsteckbar ist, die relative Orientierung der beiden Teile (1,4) zu gewährleisten, wird vorgeschlagen, daß der Boden (3) der Aufnahmeöffnung (2) und die diesem gegenüberliegende Endfläche (7) des Einsteckendes (5) mindestens je einen exzentrisch angeordneten Vor- bzw. Rücksprung (8,9) aufweisen, die bei in die Aufnahmeöffnung (2) eingesetztem Einsteckende (5) ineinandergreifen. Außerdem wird ein Bausatz für eine Endoprothese mit mehreren derartigen Verankerungsteilen (1) und mehreren derartigen Verbindungsteilen (4) beschrieben.

## Beschreibung

Die Erfindung betrifft eine Endoprothese mit einem Verankerungsteil und einem Verbindungsteil, bei der das Verbindungsteil mit einem zapfenförmigen Einsteckende in eine sacklochförmige Aufnahmeöffnung am Verankerungsteil einsteckbar ist.

Derartige Endoprothesen werden beispielsweise im Bereich der Hüfte eingesetzt, das Verankerungsteil bildet dann einen in den Markraum des Femurs einschiebbaren Schaft aus, das Verbindungsteil dient zur Verbindung dieses Schaftes mit einer Gelenkkugel. Häufig wird das Verbindungsteil als Doppelsteckkonus ausgebildet, dabei kann der Querschnitt des Einsteckendes und der Aufnahmeöffnung beispielsweise kreisförmig sein (EP 0 201 407 B1) oder länglich-oval (EP 0 310 566 B1). Bei bekannten Endoprothesen dieser Art wird der Einsteckzapfen häufig von einem Kragen umgeben, der formschlüssig in eine entsprechende Ausnehmung am oberen Ende der Aufnahmeöffnung eingreift, so daß dadurch eine definierte Position des Einsteckendes in der Aufnahmeöffnung garantiert wird, soweit es die Drehung des Einsteckendes um seine Längsachse angeht (EP 0 201 407 B1, EP 0 797 964 A1). Es sind auch Ausgestaltungen bekannt, bei denen ein zylindrisches Einsteckende des Verbindungsteils eine seitliche Orientierungsrippe trägt, die in eine entsprechende seitliche Ausnehmung der Aufnahmeöffnung eingreift (US 4,963,161).

Die Herstellung dieser Verdrehsicherungen ist relativ kompliziert und führt außerdem dazu, daß ein Teil der Seitenfläche der Steckverbindung für Elemente der Verdrehsicherung benötigt wird, dadurch kann die erwünschte sichere Abstützung des Einsteckendes in der Aufnahmeöffnung beeinträchtigt werden.

Es ist Aufgabe der Erfindung, eine gattungsgemäße Endoprothese so auszugestalten, daß in einfacher Weise ohne Modifikation der Seitenwände von Einsteckende und Aufnahmeöffnung eine definierte Einsteckorientierung sichergestellt ist.

Diese Aufgabe wird bei einer Endoprothese der eingangs beschriebenen Art erfindungsgemäß dadurch gelöst, daß der Boden der Aufnahmeöffnung und die diesem gegenüberliegende Endfläche des Einsteckendes mindestens je einen exzentrisch angeordneten Vor- bzw. Rücksprung aufweisen, die bei in die Aufnahmeöffnung eingesetztem Einsteckende ineinandergreifen. Nur bei zutreffender Relativorientierung von Einsteckende und Aufnahmeöffnung kann ein solches Ineinandergreifen erreicht werden, da sowohl Vorsprung als auch Rücksprung exzentrisch angeordnet sind, also gegenüber der Längsachse des Einsteckendes bzw. der Aufnahmeöffnung versetzt sind. Bei einer Fehlorientierung beim Einstecken stößt der Vorsprung des einen Teils an die gegenüberliegende Fläche des anderen und läßt ein vollständiges Einschieben des Einsteckendes in die Aufnahmeöffnung nicht zu. Der Operateur kann also das Einsteckende in die Aufnahmeöffnung nur dann vollständig einschieben, wenn das Einsteckende relativ zur Aufnahmeöffnung richtig orientiert ist und wenn dadurch Vorsprung und Rücksprung ineinandergreifen können.

Bei einer bevorzugten Ausführungsform ist vorgesehen, daß der Vorsprung an der Endfläche des Einsteckendes und der Rücksprung im Boden der Aufnahmeöffnung angeordnet sind. Grundsätzlich wäre aber auch die umgekehrte Anordnung möglich, bei der ein Vorsprung am Boden und ein korrespondierender Rücksprung an der Endfläche des Einsteckendes vorgesehen werden.

Eine besonders einfache Ausgestaltung ergibt sich, wenn Vor- und Rücksprung durch eine stufenförmige Ausbildung der Endfläche des Einsteckendes und des Bodens der Aufnahmeöffnung gebildet sind.

Bei einer anderen bevorzugten Ausführungsform ist vorgesehen, daß der Vorsprung die Form eines Stiftes oder Zapfens aufweist.

Der Rücksprung kann die Form einer Bohrung aufweisen. Insbesondere kann diese Bohrung als Innengewindebohrung ausgebildet sein, wenn sie im Boden der Aufnahmeöffnung angeordnet ist, dann kann diese Innengewindebohrung zusätzlich dazu dienen, mit dem Verankerungsteil ein Einsetzwerkzeug durch Einschrauben zu verbinden, es ist auf diese Weise möglich, eine zusätzliche Aufnahmebohrung für ein Einsetzwerkzeug, wie sie sonst häufig bei Verankerungsteilen notwendig wird, zu vermeiden. Der Innengewindebohrung kommt somit eine Doppelfunktion zu, nämlich einmal die Aufnahme des Handhabungswerkzeuges, zum anderen die Aufnahme eines Vorsprungs am Einsteckende des Verbindungsteils und damit eine Sicherung für die zutreffende Orientierung des Verbindungsteils im Verankerungsteil.

Das Zusammenspiel von Vorsprung und Rücksprung an den einander zugewandten Endflächen von Einsteckende und Aufnahmeöffnung dient aber nicht nur der zutreffenden Orientierung beim Einführen des Einsteckendes in die Aufnahmeöffnung, sondern es ist auch möglich, auf diese Weise die Zuordnung von unterschiedlichen Verankerungsteilen und Verbindungsteilen zu kontrollieren.

Die Erfindung bezieht sich daher auch auf einen Bausatz für eine Endoprothese, die mehrere Verankerungsteile und mehrere Verbindungsteile mit den voranstehend beschriebenen Merkmalen aufweist.

Um hier die Zuordnung von verschiedenen Verbindungsteilen zu verschiedenen Verankerungsteilen sicherzustellen, wird erfindungsgemäß vorgesehen, daß Vorsprung und Rücksprung bei verschiedenen Teilen des Bausatzes an verschiedenen Stellen am Boden der Aufnahmeöffnung und an der Endfläche des Einsteckendes angeordnet sind, so daß nur bei bestimmten Kombinationen eines Verankerungsteiles und eines Verbindungsteiles beim Zusammenstecken ein Eingriff eines Vorsprunges und eines Rücksprunges auftritt.

Nur bei Kombinationen von bestimmten Verbindungsteilen und Verankerungsteilen ist daher ein vollständiges Zusammenstecken möglich, bei allen anderen Kombinationen findet der Vorsprung keinen entsprechenden Rücksprung und stößt daher an der gegenüberliegenden Endfläche des jeweils anderen Teiles an, bevor die volle Einstecktiefe erreicht ist, dies signalisiert dem Operateur sofort, daß diese beiden Teile nicht zusammenpassen.

Eine eineindeutige Zuordnung ergibt sich beispielsweise, wenn komplementäre Vor- und Rücksprünge in unterschiedlichen Positionen und exzentrisch angeordnet sind, dadurch erhält man einerseits eine klare Auswahl von zueinander passenden Komponenten und zum anderen eine definierte Orientierungshilfe beim Einsetzen. Nur in der richtigen Orientierung und bei der richtigen Kombination kann ein Eingriff von Vorsprung und Rücksprung erzielt werden, alle anderen Orientierungen und Kombinationen scheiden also aus.

Besonders vorteilhaft ist es, wenn an einem Teil des Bausatzes in mehreren Positionen ein Rücksprung angeordnet ist, so daß dieses Teil mit Teilen kombinierbar ist, bei denen der Vorsprung unterschiedlich angeordnet ist. Es gibt also in diesem Fall keine eineindeutige Zuordnung mehr, sondern ein Teil des Bausatzes kann mit mehreren anderen Teilen dadurch kombiniert werden, daß das eine Teil in unterschiedlichen Positionen einen Rücksprung aufweist, so daß unterschiedlich angeordnete Vorsprünge aufgenommen werden können. Es ist weiterhin auch möglich, daß an einem Teil des Bausatzes in mehreren Positionen ein Vorsprung angeordnet ist, so daß dieses Teil nur mit Teilen kombinierbar ist, bei denen ein Rücksprung an mehreren, mit den Positionen der Vorsprünge übereinstimmenden Positionen angeordnet ist. Auf diese Weise läßt sich eine sehr genaue und differenzierte Zuordnung von zueinander passenden Teilen gewährleisten, nur wenn ein Vorsprung an mehreren Positionen auch einen entsprechenden Rücksprung an diesen Positionen findet, passen die Teile zueinander, alle anderen Teile werden durch diese Passung "zurückgewiesen".

Bei einer ersten bevorzugten Ausführungsform kann ein gemeinsamer Rücksprung vorgesehen sein, der sich über mehrere mögliche Positionen eines Vorsprunges am anderen Teil erstreckt. Es wird also ein Rücksprung verwendet, der eine unterschiedliche Größe hat, es kann sich dabei um eine Erstreckung parallel zum Boden der Aufnahmeöffnung oder zur Endfläche des Einsteckendes handeln, grundsätzlich wäre es aber auch möglich, daß dieser Rücksprung eine unterschiedliche Tiefe aufweist, so daß Vorsprünge unterschiedlicher Länge aufgenommen werden können, jedoch nicht Vorsprünge, deren Länge die Tiefe des Rücksprunges übersteigt. Die Angabe "mehrere mögliche Positionen" umfaßt also sowohl unterschiedliche Positionen quer zur Einsteckrichtung also auch in Einsteckrichtung.

Es ist auch möglich, daß in mehreren Positionen angeordnete Rücksprünge voneinander getrennt sind, daß also über den Boden der Aufnahmeöffnung oder die Endfläche des Einsteckendes mehrere derartige Rücksprünge verteilt sind.

Ebenso kann vorgesehen sein, daß in mehreren Positionen angeordnete Vorsprünge voneinander getrennt sind, daß also mehrere Vorsprünge nebeneinander angeordnet sind.

Es wäre aber auch möglich, daß ein gemeinsamer Vorsprung vorgesehen ist, der sich über mehrere mögliche Positionen eines Rücksprunges am anderen Teil erstreckt, und auch hier ist dies wieder sowohl quer zur Einsteckrichtung als auch in Einsteckrichtung zu verstehen.

Die Auswahlmöglichkeit der unterschiedlichen Kombinationen durch unterschiedlich tiefe Vor- und Rücksprünge wäre grundsätzlich auch bei zentral angeordneten Vor- und Rücksprüngen möglich, allerdings fehlt dann die Orientierungssicherung, durch die eine richtige Orientierung des Einsteckendes in der Aufnahmeöffnung gewährleistet wird.

Grundsätzlich kann die beschriebene Ausgestaltung einer Endoprothese oder eines Bausatzes für eine Endoprothese für Endoprothesen der unterschiedlichsten Bauart Verwendung finden, bei der ein Verankerungsteil mit einem Verbindungsteil verbunden wird. Besonders vorteilhaft ist es jedoch, wenn das Verankerungsteil der Schaft einer Femurprothese ist.

Es ist weiterhin günstig, wenn das Einsteckende und die Aufnahmeöffnung konisch ineinander passend ausgebildet sind, man erhält dann bei entsprechend geringer Konizität beim Einschieben einen selbsthemmenden Klemmsitz.

Das Einsteckende und die Aufnahmeöffnung können im Querschnitt länglich-oval ausgebildet sein, wobei hier unterschiedliche Formgebungen denkbar sind, beispielsweise eine Formgebung mit parallelen Seitenwänden, die über bogenförmige Abschnitte ineinander übergehen.

Das Verbindungsteil kann an seinem dem Einsteckende gegenüberliegenden Ende eine Aufnahme für eine Gelenkkugel aufweisen, insbesondere ist das Verbindungsteil als Doppelkonus ausgebildet.

Die nachfolgende Beschreibung bevorzugter Ausführungsform der Erfindung dient im Zusammenhang mit der Zeichnung der näheren Erläuterung. Es zeigen:
- Figur 1:: eine seitliche Teilschnittansicht eines Femurschaftes mit eingesetztem, doppelkonischem Verbindungsteil mit einem stiftförmigen Orientierungsvorsprung;
- Figur 2:: eine vergrößerte Seitenansicht des doppelkonischen Verbindungsteils der Figur 1;
- Figur 3:: eine Ansicht der Endfläche des Einsteckendes des Verbindungsteils der Figur 2 in Richtung des Pfeiles A gesehen;
- Figuren 4a bis 4d:: Ansichten von unterschiedlichen Ausführungsbeispielen der Aufnahmeöffnung für das Verbindungsteil mit jeweils einem Rücksprung im Boden in unterschiedlicher Position;
- Figuren 5a bis 5c: eine Ansicht ähnlich der Figuren 4a bis 4d mit einer unterschiedlichen Anzahl von Rücksprüngen;
- Figur 6:: eine Ansicht ähnlich Figur 2 eines Verbindungsteils mit einer stufenförmigen Endfläche des Einsteckendes und
- Figur 7:: eine Ansicht des Verbindungsteils der Figur 6 in Richtung des Pfeiles B.

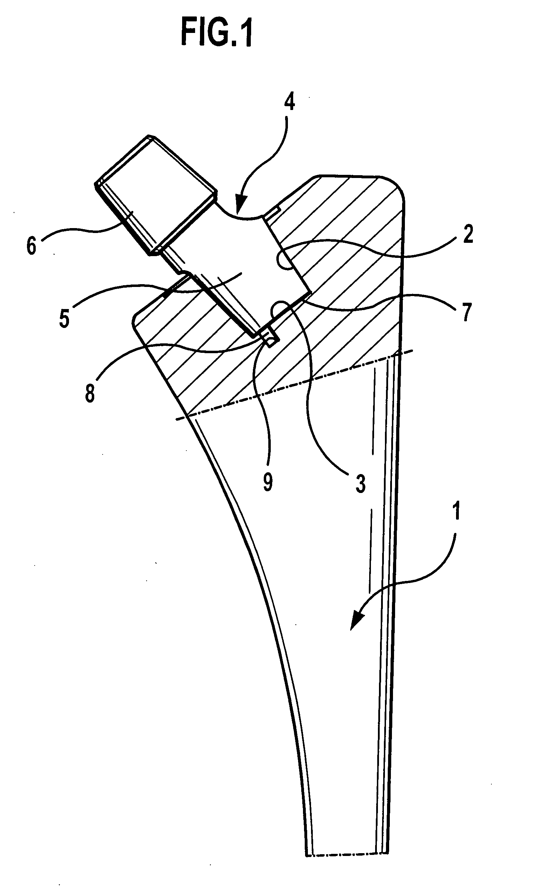

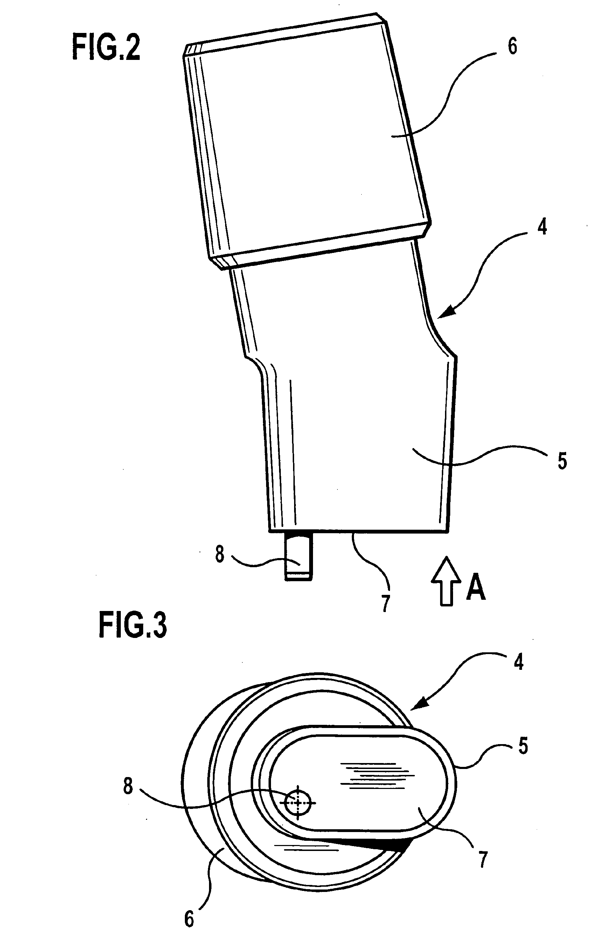

Die in den Figuren 1 bis 3 dargestellte Endoprothese umfaßt einen als Verankerungsteil wirkenden Schaft 1, der in der Zeichnung nur im oberen Teil dargestellt ist und der in an sich bekannter Weise in den Markraum eines Femurs einführbar ist. Der Schaft 1 verbreitert sich in seinem oberen Teil und weist dort eine sich zur Oberseite hin konisch öffnende Aufnahmeöffnung 2 auf, deren Längsachse gegenüber der Längsachse des Schaftes 1 geneigt ist. Die Aufnahmeöffnung 2 wird von einem ebenen Boden 3 begrenzt, im Querschnitt ist diese Aufnahmeöffnung 2 länglich-oval ausgebildet, so daß die Seitenwand zwei parallele, ebene Bereiche aufweist, die durch kreisbogenförmige Abschnitte miteinander verbunden sind.

Die Endoprothese umfaßt weiterhin ein Verbindungsteil 4, das an einem Ende als Einsteckende 5 ausgebildet ist. Dieses Einsteckende 5 ist komplementär zur Aufnahmeöffnung 2 ausgebildet und paßt somit exakt in die Aufnahmeöffnung 2 hinein, beim vollständigen Einschieben des Einsteckendes 5 in die Aufnahmeöffnung 2 liegen die Seitenflächen des Einsteckendes 5 flächig an den Seitenflächen der Aufnahmeöffnung 2 an, durch die relativ geringe Konizität von Aufnahmeöffnung 2 und Einsteckende 5 ergibt sich dabei eine selbsthemmende Klemmverbindung.

An seiner dem Einsteckende 5 gegenüberliegenden Seite trägt das Verbindungsteil 4 einen im Querschnitt kreisförmigen Aufsteckkonus 6, auf den eine in der Zeichnung nicht dargestellte Gelenkkugel in an sich bekannter Weise aufgesteckt werden kann.

Das Einsteckende 5 endet in einer dem Boden 3 gegenüberliegenden Endfläche 7, und von dieser Endfläche 7 steht in einer von der Mittelachse des Einsteckendes 5 beabstandeten Position, also exzentrisch, ein stiftförmiger Vorsprung 8 ab, der in einen ihm direkt gegenüberliegenden, sacklochförmigen Rücksprung 9 im Boden 3 der Aufnahmeöffnung 2 eintaucht.

Durch die exzentrische Anordnung von Vorsprung 8 und Rücksprung 9 ist sichergestellt, daß das Einsteckende 5 nur in einer einzigen Position in die Aufnahmeöffnung 2 eingesetzt werden kann, wenn das Einsteckende 5 um 180° verdreht in die Aufnahmeöffnung 2 eingesetzt wird, stößt der Vorsprung 8 in einem Bereich auf den Boden 3 der Aufnahmeöffnung 2 auf, der keinen Rücksprung aufweist, und damit ist das vollständige Einschieben des Einsteckendes 5 in die Aufnahmeöffnung 2 verhindert.

Der exzentrisch angeordnete Vorsprung 8 und der ihm direkt gegenüberliegende Rücksprung 9 sichern also das orientierungsrichtige Einsetzen des Einsteckendes 5 in die Aufnahmeöffnung 2.

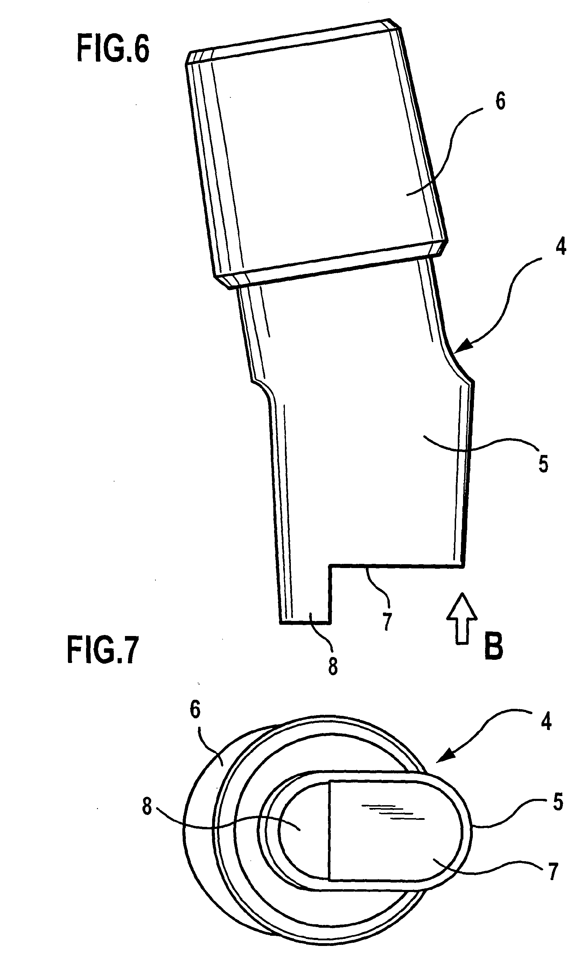

Bei dem Ausführungsbeispiel der Figuren 6 und 7 ist eine ähnliche Ausgestaltung des Verbindungsteils 4 gewählt, jedoch ist der Vorsprung 8 nicht in Form eines Stiftes ausgebildet, sondern ergibt sich durch eine stufenförmige Ausgestaltung der Endfläche 7. In entsprechender Weise ist der Boden 3 der Aufnahmeöffnung 2 stufig ausgebildet, so daß der stufige Vorsprung 8 in einen stufigen Rücksprung 9 eintauchen kann, wenn das Einsteckende 5 orientierungsrichtig in die Aufnahmeöffnung 2 eingeschoben wird, bei falscher Orientierung stößt dagegen der stufenförmige Vorsprung 8 auf den Boden 3 auf und verhindert ein vollständiges Einschieben.

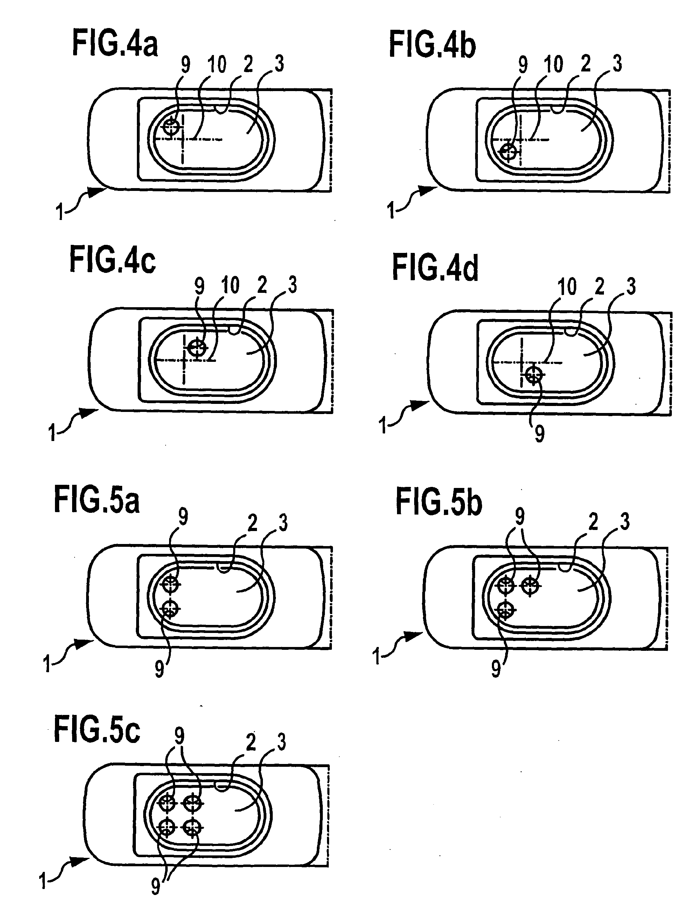

In den Figuren 4a bis 4d ist dargestellt, daß der Rücksprung 9 in Form einer Bohrung im Boden 3 der Aufnahmeöffnung 2 in unterschiedlicher exzentrischer Position angeordnet werden kann, in den Figuren 4a bis 4d sind dabei Anordnungen in jeweils einem der vier Quadranten eines gedachten Achsenkreuzes 10 dargestellt.

In entsprechender Anordnung können stiftförmige Vorsprünge 8 an unterschiedlichen Verbindungsteilen 4 angeordnet sein, beispielsweise Verbindungsteilen 4, bei denen der Winkel zwischen der Achse des Aufsteckkonus 6 und der Achse des Einsteckendes 5 unterschiedlich ist oder bei denen der Abstand von Aufsteckkonus 6 und Einsteckende 5 unterschiedlich ist. Nur solche Teile können vollständig zusammengesteckt werden, bei denen Vorsprung 8 und Rücksprung 9 unmittelbar einander gegenüberliegen, bei allen anderen Kombinationen wird das vollständige Einschieben durch das Aufsitzen des Vorsprunges 8 auf dem Boden 3 verhindert. Damit ist eine Zuordnung sichergestellt, die gewährleistet, daß ein bestimmter Schaft mit einer bestimmten Anordnung eines Rücksprunges 9 im Boden 3 immer nur mit solchen Verbindungsteilen 4 kombiniert werden kann, bei denen der stiftförmige Vorsprung 8 dem Rücksprung 9 unmittelbar gegenüberliegt, alle anderen Einsteckenden 5 werden "zurückgewiesen", da sie sich nicht vollständig einschieben lassen.

Während bei dem Ausführungsbeispiel der Figuren 4a bis 4d jeweils nur ein einziger Rücksprung 9 in unterschiedlichen Positionen vorgesehen ist, ist es auch möglich, im Boden 3 mehr als nur einen Rücksprung 9 vorzusehen, und diese Rücksprünge 9 dann den Vorsprüngen 8 in unterschiedlichen Verbindungsteilen 4 gegenüberliegend anzuordnen. Beim Beispiel der Figur 5a sind zwei derartige Rücksprünge vorgesehen, beim Beispiel der Figur 5b drei und beim Beispiel der Figur 5c vier, die Rücksprünge 9 liegen dabei nebeneinander in unterschiedlichen Positionen. Kombiniert man diese Schäfte mit Verbindungsteilen 4, die an unterschiedlichen Stellen einen oder aber auch mehrere Vorsprünge 8 tragen, dann läßt sich eine sehr selektive Zuordnung erreichen. In einen Schaft gemäß Figur 5a lassen sich beispielsweise nur Einsteckenden 5 einschieben, die jeweils nur einen Vorsprung 8 einem der beiden Rücksprünge 9 gegenüberliegend aurweisen oder die zwei Vorsprünge 8 aufweisen, die beide jeweils einem Rücksprung 9 gegenüberliegen.

Diese Verbindungsteile 4 würden auch in Schäfte einsetzbar sein, die gemäß Figur 5b oder 5c ausgebildet sind, diese Schäfte können aber zusätzlich noch Einsteckenden 5 aufnehmen, die jeweils einen oder zwei Vorsprünge gegenüberliegend den jeweiligen Rücksprüngen im Boden aufweisen oder sogar drei derartige Vorsprünge (im Beispiel der Figur 5b) oder vier derartige Vorsprünge (im Beispiel der Figur 5c). Man erhält dadurch eine vielfältige Codierungsmöglichkeit für mögliche Kombinationen einerseits und für ausgeschlossene Kombinationen andererseits.

Diese verschiedenen Codierungsmöglichkeiten sind am Beispiel der Figuren 4a bis 4d und 5a bis 5c für stiftförmige Vorsprünge und bohrungsförmige Rücksprünge erläutert worden, es versteht sich, daß dies auch für stufenförmige Ausgestaltungen der Vorsprünge und Rücksprünge gilt, in diesem Falle ist die Überdeckung der Vorsprünge 8 bzw. der Rücksprünge 9 unterschiedlich, es werden also die Einzelvorsprünge und Einzelrücksprünge der Ausführungsbeispiele der Figuren 4a bis 5c ersetzt durch Vorsprünge mit unterschiedlicher Flächenausdehnung, die Vorsprünge 8 und Rücksprünge 9 der Ausführungsbeispiele der Figuren 4a bis 5c verschmelzen also sozusagen zu unterschiedlich großen Vorsprüngen bzw. Rücksprüngen.

## Patentansprüche

1. Endoprothese mit einem Verankerungsteil und einem Verbindungsteil (4), bei der das Verbindungsteil (4) mit einem zapfenförmigen Einsteckende (5) in eine sacklochförmige Aufnahmeöffnung (2) am Verankerungsteil einsteckbar ist, **dadurch gekennzeichnet, daß** der Boden (3) der Aufnahmeöffnung (2) und die diesem gegenüberliegende Endfläche (7) des Einsteckendes (5) mindestens je einen exzentrisch angeordneten Vor- bzw. Rücksprung (8; 9) aufweisen, die bei in die Aufnahmeöffnung (2) eingesetztem Einsteckende (5) ineinandergreifen.

2. Endoprothese nach Anspruch 1, **dadurch gekennzeichnet, daß** der Vorsprung (8) an der Endfläche (7) des Einsteckendes (5) und der Rücksprung (9) im Boden (3) der Aufnahmeöffnung (2) angeordnet sind.

3. Endoprothese nach Anspruch 1 oder 2, **dadurch gekennzeichnet, daß** Vor- und Rücksprung (8; 9) durch eine stufenförmige Ausbildung der Endfläche (7) des Einsteckendes (5) und des Bodens (3) der Aufnahmeöffnung (2 ) gebildet sind.

4. Endoprothese nach einem der Ansprüche 1 oder 2, **dadurch gekennzeichnet, daß** der Vorsprung (8) die Form eines Stiftes oder Zapfens aufweist.

5. Endoprothese nach einem der Ansprüche 1 und 2 oder 4, **dadurch gekennzeichnet, daß** der Rücksprung (9) die Form einer Bohrung aufweist.

6. Endoprothese nach Anspruch 5, **dadurch gekennzeichnet, daß** die Bohrung als Innengewindebohrung ausgebildet ist.

7. Endoprothese nach einem der voranstehenden Ansprüche, **dadurch gekennzeichnet, daß** das Verankerungsteil der Schaft (1) einer Femurprothese ist.

8. Endoprothese nach einem der voranstehenden Ansprüche, **dadurch gekennzeichnet, daß** das Einsteckende (5) und die Aufnahmeöffnung (2) konisch ineinanderpassend ausgebildet sind.

9. Endoprothese nach einem der voranstehenden Ansprüche, **dadurch gekennzeichnet, daß** das Einsteckende (5) und die Aufnahmeöffnung (2) im Querschnitt länglich-oval ausgebildet sind.

10. Endoprothese nach einem der voranstehenden Ansprüche, **dadurch gekennzeichnet, daß** das Verbindungsteil (4) an seinem dem Einsteckende (5) gegenüberliegenden Ende eine Aufnahme (6) für eine Gelenkkugel aufweist.

11. Bausatz für eine Endoprothese nach einem der Ansprüche 5 bis 10 mit mehreren Verankerungsteilen und mehreren Verbindungsteilen, **dadurch gekennzeichnet, daß** Vorsprung und Rücksprung (8; 9) bei verschiedenen Teilen (4; 1) des Bausatzes an verschiedenen Stellen am Boden (3) der Aufnahmeöffnung (2) und an der Endfläche (7) des Einsteckendes (5) angeordnet sind, so daß nur bei bestimmten Kombinationen eines Verankerungsteils (1) und eines Verbindungsteils (4) beim Zusammenstecken ein Eingriff eines Vorsprunges (8) und eines Rücksprunges (9) auftritt.

12. Bausatz nach Anspruch 11, **dadurch gekennzeichnet, daß** an einem Teil (1) des Bausatzes in mehreren Positionen ein Rücksprung (9) angeordnet ist, so daß dieses mit Teilen (4) kombinierbar ist, bei denen der Vorsprung (8) unterschiedlich angeordnet ist.

13. Bausatz nach Anspruch 12, **dadurch gekennzeichnet, daß** an einem Teil (4) des Bausatzes in mehreren Positionen ein Vorsprung (8) angeordnet ist, so daß dieses Teil (4) nur mit Teilen (1) kombinierbar ist, bei denen ein Rücksprung (9) an mehreren, mit den Positionen der Vorsprünge (8) übereinstimmenden Positionen angeordnet ist.

14. Bausatz nach einem der Ansprüche 12 oder 13, **dadurch gekennzeichnet, daß** ein gemeinsamer Rücksprung (9) vorgesehen ist, der sich über mehrere mögliche Positionen eines Vorsprunges (8) am anderen Teil (4) erstreckt.

15. Bausatz nach einem der Ansprüche 12 oder 13, **dadurch gekennzeichnet, daß** in mehreren Positionen angeordnete, voneinander getrennte Rücksprünge (9) vorgesehen sind.

16. Bausatz nach einem der Ansprüche 13 bis 15, **dadurch gekennzeichnet, daß** in mehreren Positionen angeordnete, voneinander getrennte Vorsprünge (8) vorgesehen sind.

17. Bausatz nach einem der Ansprüche 13 oder 14, **dadurch gekennzeichnet, daß** ein gemeinsamer Vorsprung (8) vorgesehen ist, der sich über mehrere mögliche Positionen eines Rücksprunges (9) am anderen Teil (1) erstreckt.
